# EUROPÄISCHE PATENTSCHRIFT

(11) **EP 0 932 410 B1**
(45) Veröffentlichungstag und Bekanntmachung des Hinweises auf die Patenterteilung: **26.05.2004**
(21) Anmeldenummer: 97944894.1
(22) Anmeldetag: 25.09.1997
(51) Int. Cl.: A61K 35/78, A61P 37/04

(54) **MISTEL-(VISCUM)-EXTRAKTE**
MISTLETOE (VISCUM) EXTRACTS
EXTRAITS DE GUI (VISCUM)

(30) Priorität: 25.09.1996 DE 19639375
(43) Veröffentlichungstag der Anmeldung: 04.08.1999
(73) Patentinhaber: aar Pharma GmbH & Co. KG, 42853 Remscheid (DE)
(72) Erfinder: RUEPP, Michel, O., D-42855 Remscheid (DE)
(74) Vertreter: Jönsson, Hans-Peter, Dr.Dipl.-Chem.
(86) Internationale Anmeldenummer: PCT/EP1997/005271
(87) Internationale Veröffentlichungsnummer: WO 1998/013053

(56) Entgegenhaltungen:
- EP-A- 0 602 686
- WO-A-97/11967
- US-A- 5 547 674
- DATABASE WPI Section Ch, Week 8850 Derwent Publications Ltd., London, GB; Class B04, AN 88-358885 XP002053152 & RO 94 491 A (INTR MEDICAMENTE BIOFARM) , 30.Juni 1988
- PATENT ABSTRACTS OF JAPAN vol. 016, no. 375 (C-0973), 12.August 1992 & JP 04 120019 A (TAKAO MATSUNO;OTHERS: 01), 21.April 1992,
- DATABASE WPI Section Ch, Week 9719 Derwent Publications Ltd., London, GB; Class B04, AN 97-211058 XP002053153 & RO 110 907 B (PLANTAVOREL SC CCPPM SA) , 30.Mai 1996

## Beschreibung

Gegenstand der Erfindung ist die Verwendung von (Viscum album) Mistel-Trockenextrakten zur Herstellung oral applizierbarer Arzneimittel.

Aus Römpp Chemielexikon, 9. Auflage, S. 2808, (1991) ist zu entnehmen, daß die Mistel ein einheimischer Vertreter der etwa 1100 Arten umfassenden, meist in den Tropen vorkommenden Mistelgewächse ist, der als immergrüner Halbschmarotzer auf verschiedenen Bäumen vorkommt, beispielsweise auf Linde, Pappel, Tanne, Kiefer, Apfel, Eiche und dort, insbesondere in winterlich entlaubten Bäumen als circa 25 bis 100 cm großes mehr oder weniger kugeliges Gewächs aufwächst. Misteln enthalten neben Viskotoxin Acetylcholin, Cholin und Lektine, Flavonoide, Lingane, Inosit, Saponine und Phenylpropan-Derivate.

Die Mistel ist als Arznei- und Zaubermittel schon seit altersher in Gebrauch. Mistelextrakte und Herba Visci in Teemischungen werden auch heute noch als blutdrucksenkende, Cholin-ähnlich wirkende Mittel sowie vor allem bei Arthrosen und rheumatischen Erkrankungen verwendet. Die (umstrittene) Wirkung von parenteral applizierten Mistelextrakten als Krebsheilmittel ist auf Protein-Fraktionen, die Glykoproteide enthalten, zurückzuführen.

Der Stand der Technik zeigt, daß die Mistel überwiegend in der Form von standardisierten, parenteral applizierbaren Präparaten auf der Basis von Lektinkonzentraten aus Mistelextrakten zubereitet wird und in der Tumor-Therapie Verwendung findet. Demgegenüber besteht die Aufgabe der vorliegenden. Erfindung in der Bereitstellung eines oral applizierbaren Arzneimittels.

Die vorstehend genannte Aufgabe wird erfindungsgemäß gelöst durch die Verwendung von standardisierten Mistel-(Viscum album)-Trockenextrakten mit einem Droge-Extrakt-Verhältnis (DEV nativ) von 4 zu 1 bis 7 zu 1, entsprechend 20 zu 1 bis 35 zu 1 bei der Frischpflanze zur Herstellung von oral applizierbaren Arzneimitteln zur Behandlung von allgemein immunsupprimierten Zuständen und zur zellulären Immunstimulation.

Weitere bevorzugte Verwendungen werden in den Unteransprüchen ausgestaltet. So betrifft die Erfindung in einer bevorzugten Ausführungsform die Verwendung nach Anspruch 1 zur Behandlung von strahlen-,infektions- und chemisch bedingten Organ- und Gewebeschädigungen, insbesondere des O-MALT-Systems des Dünndarms (entzündliche Erkrankungen, Enteritis regionalis Crohn), des Knochenmarks (Knochenmarkaplasie), des Thymus (Dysfunktion, Aplasie oder Hypoplasie), der Milz (Dysfunktion) und/oder der Lymphknoten (Aplasie oder Hypoplasie infolge medikamenten- oder strahlenbedingter Schädigung), der Leber (Atrophie, Nekrose), Hepatitis A, B und C, der Bauchspeicheldrüse (Insuffizienz der exokrinen, sekretorischen Funktion von Proteasen, Esterasen, Carbohydrasen und/oder Nukleasen sowie der endokrinen Funktionsstörung des Kohlenhydratstoffwechsels [Langerhanssche Inseln]) und/oder der Nieren (Insuffizienz) sowie von allgemein immunsupprimierten Zuständen, zur zellulären Immunstimulation, zur Therapie von Leukozytopenie, Granulozytopenie, Lymphozytopenie, Thrombozytopenie, Erythrozytopenie und bei Immunglobulinmangelzuständen, auch infolge von AIDS und Tumoren sowie zur Therapie von Hyperlipoproteinämien, Hyperproteinämie und Hpyerlipidämie.

Die zelluläre Immunstimulation bewirkt dabei eine Zunahme der Zellzahlen von Leukozyten, Thrombozyten, Erythrozyten, segmentkernigen Granulozyten und Lymphozyten, T-, B-, Helfer-, Suppressor- und NK-Zellen, insbesondere von B-Lymphozyten in der terminalen Strombahn, so daß eine Zunahme vitaler Lymphozyten in der Rindenzone und den -follikeln in den Peyersche Plaques (Dünndarm) zu erkennen ist, die mit einer sekretorischen Induktion von sIGA in den Dünndarm verbunden ist, daß eine Zunahme aller Blutbildungselemente im Knochenmark feststellbar ist, daß eine zelluläre Stimulation immunkompetenter Zellen in Thymus, Milz und mesenterialen Lymphknoten nachweisbar ist und/oder daß die Stoffwechselfunktionen von Leber, Pankreas und Nieren (Senkung der Serumwerte von Kreatinin, Harnsäure, Glukose, GOT, GPT und GGT) deutlich aktiviert werden.

Gegenstand der Erfindung ist insbesondere ein oral applizierbarer Mistel-(Viscum)-Trockenextrakt in Kombination mit Echinacea-, Cynara- und/oder Urtica-Extrakten.

Eine erfindungsgemäße Verwendung der beschriebenen Mistel-(Viscum album)-Extrakte betrifft auch die Herstellung eines entsprechenden Arzneimittels gemäß der Unteransprüche zur Behandlung von Störungen des Kohlenhydratstoffwechsels und/oder der Leber- und Nierenfunktion, zur Behandlung von prädiabetischen, insbesondere senilen Verlaufsformen, zur Behandlung von latenter diabetischer Stoffwechsellage, insbesondere bei Schwangerschaft, zur Behandlung von Infektionen, Streß und/oder Fettleibigkeit, zur Behandlung von Bluthochdruck als synergistisches Adjuvanz nach simultaner Applikation mit chemisch definierten Antihypertonika und als adjuvante Therapie der Diabetes mellitus bei vorhandener Restfunktion der Pankreas.

Erfindungsgemäße Verwendungen gemäß der Unteransprüche betreffen auch orale Adjuvanzien, die der malignen Tumorbehandlung, - insbesondere der Behandlung von Mamma-, Portio-, Kolon- oder Prostatakarzinomen dienen, auch in Kombination mit Cytostatika, insbesondere Cyclophosphamid oder Strahlentherapie -, der Behandlung von Erkrankungen mit supprimiertem Immunsystem, insbesondere AIDS.

Auch zur Steigerung der Verträglichkeit von chemo-, zytostatischer, radiologischer Therapie, insbesondere in Kombination mit chemisch definierten Substanzen, ausgewählt aus Clofibrat, ACE-Hemmern, a-2-Rezeptor-Antagonisten, Ca-Antagonisten und/oder Diuretika, als Adjuvanz der Analgesie, insbesondere als pharmakologische Komponente der positiven Beeinflussung der Wechselwirkungen zwischen Endokrinum, Nerven- und Immunsystem werden die beschriebenen Arzneimittel erfindungsgemäß verwendet.

Weiterhin dienen sie als Adujvanz bei der Behandlung von bakteriell und viral induzierten Krankheitsbildern, insbesondere entzündlichen Erkrankungen des Dünndarms (Enteritis regionalis Crohn), der Bauchspeicheldrüse und der Nieren, Leberatrophie, Hepatitis A, B und C, Hautläsionen (Ulcus cruris), wie auch Herpex simplex I und **II und Herpes zoster.**

Erfindungsgemäß konnten in einer In-vivo-Studie an Ratten mit einer Zubereitung nach peroraler Applikation aus Viscum album Trockenextrakt (VN), insbesondere von Mistelkraut (visci herba nach dem Deutschen Arzneibuch (DAB)), definierte Parameter des Kreislaufsystems einschließlich Blutdruck- sowie des Immun- und Neutrotransmittersystems beeinflusst werden.

Die verwendeten Mistel-Trockenextrakte (100 mg/kg KGW) induzierten:
- keinen von der Kontrollgruppe abweichenden Effekt der durchschnittlichen Körpergewichtsentwicklung
- keine Änderungen der durchschnittlichen Organgewichte von Milz und Thymus
- eine Zunahme immunkompetenter Zellen, nämlich von Erythrozyten, segmentkernigen Granulozyten, Thrombozyten und Leukozyten sowie der Lymphozyten, T-, B-, Helfer-, Suppressor- und NK-Zellen
- eine Erhöhung des Hämoglobingehalts, Hämatokrit- und Kaliumwertes
- eine Abnahme der Gehaltswerte von GLDH, GOT, GPT, AP und Gamma-GT sowie von Cholesterin und Triglyceriden
- eine Senkung des Plasma-Noradrenafinwertes als mögliche Komponente hypotoner und analgetischer Wirkung
- eine Abnahme systolischer normo- und hypertensiver Blutdruckwerte.

Als therapeutisch wünschenswert werden angesehen:
- die klinisch nachweisbare Abnahme des systolischen Blutdrucks
- die Senkung der Cholesterin- und Triglyzeridwerte zur Verbesserung der Fließeigenschaft des Blutes
- die Zunahme der Erythrozytenzahl, des Hämoglobingehaltes im peripheren Blut und die Zellmehrproduktion des roten Knochenmarks in der Spongiosa des Sternums als Ausdruck einer verbesserten Sauerstoffversorgung, aber auch als aktueller Nachweis einer hämostyptischen Wirkung - diese unterscheiden sich offensichtlich von der Wirkungsart lokaler und parenteraler Hämostyptika
- die Zunahme definierter immunkompetenter Zellen als Adjuvanz für eine entzündungshemmende Wirkung: stabile relative Zellzahlverhältnisse bei T-Lymphozyten, Helfer- und Suppressorzellen sowie die prozentuale gleichmäßige Zunahme aller lymphatischen Zellen - generell als Ausdruck einer physiologischen Zellzahlvermehrung
- der Rückgang der leberspezifischen Aktivitätswerte von GLDH, GOT, GPT, AP und Gamma-GT als Ausdruck eines hepatokurativen Effektes
- fehlende Anzeichen unerwünschter Wirkungen.

Aus den experimentellen Ergebnissen kann die Schlußfolgerung gezogen werden, daß die registrierten Änderungen definierter hämodynamischer, hämatologischer und klinisch-chemischer Analysenwerte im Sinne der in der Literatur beschriebenen antihypertensiven Effekte einer Misteltherapie interpretiert werden können, wie sie zum Beispiel auch mit Handelspräparaten nachgewiesen wurden.

Auch klinisch überzeugende Untersuchungsergebnisse an 120 Patienten belegen eine signifikante systolische und diastolische Blutdrucksenkung von 9 % (gemessen im Sitzen) nach sechswöchiger Behandlung mit einem Mistelextrakt-Präparat. Diese therapeutische Wirkung geht mit einer deutlichen Verbesserung kontrollierter sowie hypertoniebedingter Verlaufsparameter wie Beeinträchtigung allgemeiner Befindlichkeit, Kopfschmerz, Schwindel sowie rascher Ermüdbarkeit einher.

Die oral applizierten Mistel-Trockenextrakte-Dragees einer erfindungsgemäß behandelten Gruppe und ein handelsüblicher Extrakt zur parenteralen Applikation zeigten hinsichtlich der Zellzunahme aller lymphatischen Zellen bioäquivalente Potentiale.

Mistel-Trockenextrakte, oral verabreicht, zeigten im Vergleich mit der parenteralen Applikation von Mistelzubereitungen ein deutlich unterschiedliches Wirkprofil:
- keine Zunahme des Milz- und Thymusgewichtes
- keine selektive Hemmung bezüglich der Zellzahlzunahme spezifischer lymphatischer Zelltypen wie Suppressor-Zellen oder gar temporäre Suppression von Helfer-Zellen.

Die Mistel-Trockenextrakte zeigten somit in der vorliegenden experimentellen Studie bei der geprüften Dosierung von 100 mg/kg KGW und den zur Auswertung gelangten Parametern keine unerwünschten Wirkungen.

Die Untersuchungen wurden mit den entsprechenden standardisierten Pflanzenextrakten in calciumcarbonat- und sacchardhaltiger Granulatform durchgeführt (Hilfsstoffe für die Granulatform: Calciumcarbonat, Eisenoxid, Magnesiumstearat, Celluloseacetatphthalat, Natriumcarboxymethylcellulose, Talkum, Triacetin, Arabisches Gummi, Carnaubawachs, Saccharose, Lactose und Siliciumdioxid, Kartoffelstärke).

Gegebenenfalls können die Dragees an sich bekannte Überzüge enthalten, um einen gewünschten Auflösungsort nach der Applikation, beispielsweise im Dünndarm, zu erreichen. Beispielsweise sind Überzüge aus Schellack, Celluloseacetatphthalat, Cellulosederivate und andere bekannt.

Die Extraktion zur Herstellung der Trockenextrakte kann mit Wasser, mit organischen Lösungsmitteln oder mit überkritischem Kohlendioxid durchgeführt werden. Besonders bevorzugt im Sinne der vorliegenden Erfindung wird als Auszugsmittel der Pflanze Methanol eingesetzt.

Als Applikationsform bietet sich die Frischpflanze, insbesondere Preßsaft, in getrockneter Form, insbesondere Pulver, Granulat, insbesondere Kapsel, und als Tablette, insbesondere Dragee oder Pastille an.

### Ausführungsbeispiel 1:

### Tiere und Tierhaltung

Männliche Wistar-Ratten mit einem durchschnittlichen Körpergewicht von 310 g wurden unter konventionellen Bedingungen bei Raumtemperatur (21 ± 1°C) in einer relativen Luftfeuchtigkeit von etwa 60 % und einem 12 Std.-Tag/Nacht-Rhythmus gehalten. Sie unterlagen vor Versuchsbeginn einer Akklimatisation von 12 Tagen an diese Haltungsbedingungen.

Die Ernährung erfolgte mit einer pelletierten Standarddiät Altromin C 1000 Misch. Nr. 100 (Firma Altrogge, Lage).

Als Trinkwasser erhielten die Tiere Leitungswasser ad libidum.

### Prüfsubstanzen und Dosierung:

Drageekerngranulat mit Trockenextrakt aus Mistelkraut auf der Basis Calciumcarbonat/Saccharose
   Auszugsmittel: Gereinigtes Wasser DAB 10
   Ausgangsdroge: Mistelkraut (Herba visci) DAB 10
   1 Drageekem mit 240 mg enthielt nativen Viscumextrakt (4-7:1) 150 mg (VN);
Applikationsform: Drageekern-Granulat 1,6 mg (≅ 1 mg nativer Trockenextrakt VN wurden in 0,02 ml Aqua dest. suspendiert VNS) 0,5 ml VNS = 25 mg VN/250 mg KGW.

### Applikation:

Die Prüfsubstanzen wurden einmal täglich 7 Tage lang den nicht sedierten Tieren intragastral mit Hilfe einer starren Knopfsonde verabreicht. Die Suspension wurde unmittelbar vor ihrer Applikation frisch hergestellt und homogen appliziert. Die Kontrolltiere erhielten physioloigsche NaCl-Lösung im äquivalenten Volumen.

12 männliche Wistar-Ratten wurden zur Untersuchung in folgende Behandlungsgruppen eingeteilt:

| Gruppe: | | Dosis: (mg/kg KGW) | Tierzahl: |
|---|---|---|---|
| I Kontrollgruppe | (Vergleich) | -- | 5 |
| II VN | (Erfindung) | 100 | 5 |

### Blutentnahme:

Am 7. Behandlungstag wurde den Tieren 2 Stunden nach der Applikation 2 x 500 µl Blut aus dem retroorbitaten Venenplexus entnommen, in Natrium EDTA beschichtete Gefäße gefüllt und gut durchmischt.

Die vorliegenden Untersuchungen verfolgten das Ziel mit Hilfe eines definierten In-vivo-Modells spezifische Marker simultan zu erfassen, die die therapeutische Wirkung als synergistischen Effekt unterschiedlich beeinflußter physiologischer Regelkreise frühzeitig anzeigten.

Im einzelnen wurden im peripheren Blut erfaßt:
- kombiniert:
   Erythrozyten (RBC), Leukozyten (WBC), Thrombozyten (Plt), Hämoglobin (HGB), Hämatokrit (HCT), Erythrozytenindizes: Mittleres Zellvolumen (MCV), Mittleres korpuskuläres Hämoglobin (MCH), Mittlere Hämoglobinkonzentration der Erythrozyten (MCHC) und Erythrozytenverteilungsbreite (RDW)
- durchflußzytometrisch:
   Leukozytendifferenzierung: Granulozyten, Monozyten, Lymphozyten, B-, T-Zellen, Helfer- und Suppressor-Zellen
- säulenchromatographisch:
   Adrenalin, Noradrenalin
- kombiniert:
   GPT, GOT, AP,
   Glukose,
   Cholesterin, Triglyceride,
   Na, K, Cl, Ca,
   Kreatinin, Harnstoff, Gesamtprotein.

Mit Hilfe des vollautomatischen Hämatologie-Analysegerätes Sysmex K-1000 konnten bestimmt werden: WBC, RBC, Plt, HGB, HCT, MCV, MCH, MCHC. Die Haupteinheit dieses Gerätes bestand im wesentlichen aus einem hydraulischen (HS) und einem elektronischen System (ES). Mit Hilfe des HS wurde angesaugt, pipettiert, verdünnt, gemischt und lysiert. Das ES analysierte und wandelte die Signale des HS um und übermittelte die Ergebnisse an den Drucker. Das ES überwachte mit Hilfe von Mikroprozessoren ebenso die Testabläufe, die Teststation und führte die Qualitätskontrolle durch.

Der Hämatokritwert gab den prozentualen Volumenanteil der Erythrozyten im Blut an. Hämoglobin, das in den Erythrozyten enthaltene Chromoprotein war neben der Erythrozytenzahl und dem Hämatokritwert ein wichtiges Kriterium der Diagnostik von Anämien. Die Klassifizierung erfolgte durch die Erythrozytenindizes. Erythrozytengröße und Hämoglobingehalt wurden durch das Erythrozytenvolumen (MCV = mean corpuscular volume), den Hämoglobingehalt der Erythrozyten (MCH = mean corpuscular haemoglobin) und die mittlere korpuskuläre Hämoglobinkonzentration (MCHC = mean corpuscular haemoglobin concentration) gekennzeichnet. Die Erythrozyten-Verteilungsbreite (RDW = red cell distribution width) war ein Maß der Anisozytose.

Die Parameter wie Enzyme, Glukose, Lipide, Elektrolyte, Kreatinin, Harnstoff und Protein wurden mit Hilfe des Analysengerates Cobas Mira selektiv, methodenorientiert, photometrisch oder ionenselektiv erfaßt.

Die Leukozytendifferenzierung wurde mit Hilfe des Durchflußzytometers FACScan nach entsprechender Lysierung der Vollblutprobe durchgeführt (Streulichtmessung).

Die Lymphozytendifferenzierung erfolgte nach spezifischer monoklonaler Inkubation mittels fluoreszensaktivierter Zellorientierung (Fluoreszensmessung).

Quantitativ wurden am 7. Behandlungstag analysiert:
Leukozyten (gesamt), Lymphozyten (gesamt),
T-Lymphozyten (CD2+/CD45 RA-),
B-Lymphozyten (CD2-/CD45 RA+),
Helfer-Lymphozyten (CD4-/CD8b+),
sowie NK-Zellen (CD8a+/CD8b-).

Zur Phänotypisierung der Lymphozyten wurden diese mit folgenden Antikörpern der Fa. Pharmingen, San Diego, USA inkubiert, an welche ein Fluorochrom gekoppelt ist:
Fluorescein Isothiocyanate (FITC) conjugated Mouse Anti-Rat CD 2 Monoclonal Antibody, -Phycoerythrin (RPE) conj. Mouse Anti-Rat CD45RA OR A/B Monoclonal Antibody, Fluorescein Isothiocyanate (FITC) Mouse Anti-Rat CD4 Monoclonal Antibody, R-Phycoerytrin (RPE) conj. Mouse Anti-Rat CD 8 (β-Chain) Monoclonal Antibody, Fluorescein Isothiocyanate (FITC) conj. Mouse Anti-Rat CD8A Monoclonal Antibody.

Ansatz:
a) CD/2CD45RA = T- und B-Zellen
b) CD4/CD8b = T₄- und T₈-Zellen
c) CD8a/CD8b = NK-Zellen

Je 5 µl Antikörper wurden mit 50 µl Na-EDTA-Blut bei Zimmertemperatur im Dunkeln 20 min lang inkubiert. Die Suspension wurde mit 2 ml Lyses Reagenz der Fa. Becton-Dickinson geschüttelt und wie beschrieben 10 min lang inkubiert. 6 min lang wurde anschließend bei 400 G zentrifugiert, der Überstand abgegossen. Das Sediment wurde mit 3 ml Cell-Wash gewaschen und 6 min lang bei 400 G zentrifugiert. Das Sediment wurde in 100 µl Cell-Wash aufgenommen. Die Suspension wurde mit Hilfe des Durchflußzytometers analysiert.

Adrenalin und Noradrenalin wurden on-line mittels Phenylborsäure aus der Matrix isoliert, anschließend mit Hilfe einer lonenpaarphase auf einer HPLC-Säule getrennt, zu Trihydroxyindolen (THT) in Reaktionskapillaren derivatisiert und fluorimetrisch detektiert.

### Klinische Beobachtungen:

Während der Akklimatisation vor Versuchsbeginn und im Verlauf der gesamten Versuchsdauer wurde das Allgemeinbefinden der Tiere kontrolliert. Zusätzlich erfolgte täglich eine Bestimmung des Körpergewichts.

### Erythrozyten, Leukozyten, Thrombozyten

Das Potential der Prüfsubstanz VN wurde indirekt durch ihre Stimulationswirkung auf die Anzahl der Leukozyten (WBC), Erythrozyten (RBC) und Thrombozyten (Plt) bestimmt.

Für die Prüfsubstanz VN wurde gemessen:
- deutliche Zunahme der Leukozytenzahl um 25%¹⁾
- eine geringe Zunahme der Erythrozytenzahl um 7,2% und der Thrombozytenzahl um 7,3%.

¹⁾ entsprechende Werte der Kontrollgruppe = 100%

### Hämoglobin, Hämatokrit, Erythrozytenindizes

Die prozentuale Zunahme des Hämoglobingehaltes wie auch des Hämatokritwertes in der Behandlungsgruppe (VN) korrelierte mit der entsprechenden Zunahme der Erythrozyten.

Die Indexwerte MCV (Quotient aus Hämatokrit und Erythrozytenzahl) und MCH (Quotient aus Hämoglobin und Erythrozytenzahl) waren unter dem Einfluß der Prüfsubstanz VN hinsichtlich der prozentualen Veränderungen geringgradig niedriger, von MCHC geringgradig höher.

### Bilirubin, Kreatinin, Harnstoff, Glukose, Lipide, Elektrolyte und Eiweiß

Folgende klinisch-chemische Stoffwechsel-Meßgrößen wurden kombiniert methodenspezifisch analysiert:
Bilirubin, Kreatinin, Harnstoff, Glutamat-Oxalacetat-Transaminase (GOT), Glutamat-Pyruvat-Transaminase (GPT), Alkalische Phosphatase (AP), Gamma-Glutamyltransferase (GGT), Glukose, Cholesterin, Triglyzeride, Calcium, Natrium, Kalium, Chlorid und Gesamtprotein.

Für die Prüfsubstanz VN wurde gemessen:
- eine deutliche Abnahme von:

| | |
|---|---|
| GLDH | um 9,5% |
| GPT | um 19,4% |
| AP | um 12, 8 % |
| GGT | um 16,7% |
| Cholesterin | um 27,7 % |
| Triglyzeride | um 47,1 % |

- eine geringe Abnahme von:

| | |
|---|---|
| GOT | um 4,9% |
| Calcium | um 5,8 % |
| Chlorid | um 3,9 % |
| Protein | um 3,6 % |

- eine deutliche Zunahme von:

| | |
|---|---|
| Kalium | um 15,3% |

In der Behandlungsgruppe II wurde im Vergleich mit der Kontrollgruppe die Zellzahlen der segmentkernigen Granulozyten, Monozyten, T- und B-Lymphozyten, Helfer, Suppressor- und NK-Zellen gemessen.

Für die Prüfsubstanz VN wurde gemessen:
- Zunahme der Leukozyten in der Gruppe II um 20,4%¹⁾
- Zunahme der T-Lymphozyten in der Gruppe II um 24,6 %
- Zunahme der B-Lymphozyten in der Gruppe II um 10,4%
- Zunahme der Helfer-Zellen in der Gruppe II um 28,2 %
- Zunahme der Suppressor-Zellen in der Gruppe II um 29,4 %
- Zunahme der NK-Zellen in der Gruppe II um 101 %
- Zunahme der Monozyten in der Gruppe II um 201 %

### Bestimmung von Catecholaminen

Die Prüfsubstanz VN zeigte im Vergleich mit der Kontrollgruppe einen ausgeprägten Einfluß auf den Noradrenafingehalt. Dieser wurde in der Behandlungsgruppe II (VN) im Vergleich zur Kontrollgruppe um 60 % gesenkt. Im Gegensatz hierzu nimmt der Gehalt von Adrenalin in der Behandlungsgruppe leicht zu.

### Ausführungsbeispiel 2:

In einer offenen multizentrischen Untersuchung wurden 64 Patienten (31 Frauen, 33 Männer) im Alter von durchschnittlich 58 Jahren mit Grenzwerthypertonie, d. h. diastolischem Blutdruck (DB) 90 - 94 mm Hg ≅ 20 Patienten, milder Hypertonie, DB 95 - 104 mm Hg ≅ 32 Patienten, mittelschwerer Hypertonie, DB 105 - 114 mm Hg ≅ 12 Patienten, durchschnittlich 2,5 Monate lang mit Dragees behandelt. Die Dragees enthielten 150 mg Mistel-Trockenextrakt auf einer Basis von Calciumcarbonat und Saccharose.

Der mittlere systolische Wert konnte von 165 auf 136 mm Hg um 18,2 % und der diastolische Wert von 95 auf 80 mm Hg um 15,8 % gesenkt werden. Eine Senkung des systolischen Blutdruckes um mehr als 10 mm Hg wurde bei 56 % der Patienten beobachtet, im diastolischen Bereich bei 31%.

Das Patientenkollektiv kann retrospektiv nach der Behandlung in drei Gruppen eingeteilt werden. 25 Patienten einer Gruppe nahm vor der Behandlung kein Arzneimittel zur Senkung des Blutdrucks ein. Bei dieser lag der systolische Wert bei 172 mm Hg und der diastolische bei 99 mm Hg. Mit den Dragees konnte eine Senkung auf 143 mm Hg bzw. 78 mm Hg erreicht werden. Im systolischen Bereich wurde somit ein Rückgang um 17 % und im diastolischen Bereich um 22 % registriert.

Des weiteren wurde eine Gruppe mit Patienten gebildet, die vor ihrer Behandlung milden Dragees bereits mit β-Blockern, Calciumantagonisten bzw. ACE-Hemmern behandelt wurden. Diese Patienten litten trotz dieser Arzneimitteleinnahme unter Hypertonie. Aufgrund dieser Diagnose wurde das chemisch definierte Arzneimittel abgesetzt und durch die Dragees ersetzt. Auch hier ist eine deutliche Senkung der mittleren Blutdruckwerte zu erkennen. Der systolische Wert sank von 172 mm Hg auf 141 mm Hg (-19%), derdiastolische von 98 mm Hg auf 71 mm Hg (-28%).

9 Patienten nahmen während der Behandlung mit den Dragees zusätzlich noch zuvor verordnete Arzneimittel ein. Auch in dieser Gruppe war eine Abnahme der Blutdruckwerte zu erkennen, wobei die Kombination der Dragees mit einem chemisch definierten Antihypertonikum die Blutdruckwerte deutlicher beeinflußte als die alleinige Gabe chemisch definierter Antihypertonika. Der systolische Wert sank von 170 mm Hg auf 145 mm Hg (-15%) und der diastolische Wert von 99 mm Hg auf 90 mm Hg (-10%).

Eine Besserung der allgemeinen Befindlichkeit gaben 48 % des Patientenkollektivs an. Dazu zählen die Verminderung von Kopfschmerzen, die Abnahme einer raschen Ermüdbarkeit sowie der Symtome von Schwindel.

Bei 20 Probanden wurde ein anderes Beschwerdebild, nämlich Tinnitus, mitgeteilt. Nach einer durchschnittlichen Behandlungsdauer von 2,5 Monaten berichteten 16 Patienten (-80 %) über eine Besserung. Weitere Patienten machten Angaben zur Wirksamkeit der Dragees bei Herpes simplex. Nach der Behandlung gaben 3 Patienten an, daß die Rezidivhäufigkeit deutlich zurückgegangen sei. Abschließend sei vermerkt, daß für 2 Patienten Resultate über additive Effekte im Rahmen einer Karzinombehandlung nach oraler Applikation der Dragees vorliegen.

Der positive Einfluß der Dragees konnte auch bei den blutchemischen Werten ermittelt werden. So wurden der Cholesterinwert von 257 auf 230 mg/dl gesenkt (-11%) und der Triglyzeridwert von 196 auf 158 mg/dl (-20 %). Auch der Glukose-Spiegel im Blut fiel unter der Behandlung mit den Dragees von 102,55 auf 93,6 mg/dl (-9%). Der Harnsäure-Wert im Blut zeigte ebenfalls einen Rückgang von 6,3 auf 5,5 mg/dl (-13%).

Die Mistel-Trockenextrakt-Dragees senkten - weniger ausgeprägt als chemisch definierte Antihypertonika - erhöhte systolische und diastolische Blutdruckwerte. Diese Wirkung wurde begleitet von lipid-, glucose- und harnsäuresenkenden Effekten. Die Dragees zeigten keine negativen Begleiterscheinungen.

Die Verträglichkeit chemisch definierter Pharmaka wurde nach simultaner Applikation der Dragees begünstigt, was auch auf eine hepatokurative und analgetische Komponente zurückgeführt wird. Darüber hinaus existierte ein viscumspezifisches Potential (experimentell), das sich positiv auf den Rückgang von Schwindelstörungen, des Tinnitussyndroms und der Häufigkeit und Intensität der Kopfschmerzen auswirkte. Die bei allen Patienten deutlich ausgeprägten kurativen Effekte hinsichtlich der Befindlichkeitsstörungen wurden im Sinne einer unspezifischen Verbesserung des Immunstatus interpretiert. Patienten mit einem immunsupprimierten Status profitierten von der oralen Viscumbehandlung; wobei die Zunahme der Lymphozytenzahl mit den tierexperimentell erhobenen Daten korrelierte.

Bei einem Vergleich der Ergebnisse der tierexperimentellen mit denen der klinischen Studie imponierten gemeinsame Tendenzen, die im Falle von Cholesterin, Triglyzeriden und systolischem Blutdruck durch abnehmende Werte, im Falle von Lymphozyten und leberspezifischen Enzymen durch zunehmende Werte zum Ausdruck kamen.

In einer experimentellen Studie an Ratten wurde die schädigende Wirkung eines definierten Zytostatikums (hier Cyclophosphamid in einer Dosierung von 10 mg/kg KGW) auf das rote Knochenmark und die lymphatischen Organe, Thymus, Milz, Lymphknoten und Peyer-Plaques durch die gleichzeitige Behandlung mit den Dragees (Wirkstoff: Viscumextrakt 100 mg/kg KGW nach peroraler Applikation) deutlich reduziert.

Nach Applikation des alkylierenden Zytostatikums, welches Atrophie, blutbildende und lymphatische Zellschädigungen von Knochenmark, Thymus, Milz, mesenterialen und zervikalen Lymphknoten induziert, läßt sich histologisch eine partielle akzidentielle Regeneration nach simultaner Behandlung mit der Viscumpräparation nachweisen. Mikromorphologisch zeigen die entsprechenden Organe deutliche Anzeichen einer Restaurierung der organspezifischen Architektur und eine deutliche Wiederbesiedlung des retikulären Stromas mit intakten Zellen der myeloischen und insbesondere der lymphatischen Gruppe.

Diese akzidentielle Regeneration des blutbildenden und lymphoretikulären Gewebes läßt keine Anzeichen einer gesteigerten mitotischen Aktivität im Sinne einer pathologisch gesteigerten Proliferation erkennen.

Im Falle des Lymphknotens, zum Beispiel erkennt man histologisch eine Wiederbesiedlung des retikulären Gewebes mit Zellen der Lymphatischen Gruppe. In der parakortikalen Zone imponieren dichtgelagerte, intakte lymphatische Zellen, in der kortikalen Zone befinden sich locker gelagerte, intakte Lymphozyten mit deutlicher Tendenz zur subkapsularen Follikelbildung. Mit der Wiederherstellung der charakteristischen Mikrostruktur des lymphoretikulären Gewebes im Lymphknoten setzt offensichtlich auch dessen Funktion wieder ein.

In jüngster Zeit haben die verschiedenen Reaktionsformen des Lymphknotens eine praktische Bedeutung erlangt: wird im Drainagegebiet von Karzinomen (Mammakarzinom, Portiokarzinom) eine Stimulation der T-Zellen (Parakortikalzone) beobachtet, so besteht bei den Patienten eine bessere Prognose als bei der Aktivierung der B-Zellen-Region oder keiner Reaktion des Lymphknotens.

Die erfolgreiche Abwehr belebter äußerer Krankheitsursachen durch das körpereigene Immunsystem hängt ganz entscheidend von der Stärke und Qualität der Immunreaktion des Wirtes ab. Eine intakte, zelluläre und humorale Abwehr des Wirtsorganismus kann Entstehung und Ausbreitung eines Tumors in Schach halten. Die wesentlichste Quelle der Abwehrschwache heutzutage ist aber die zytostatische Kortikoidtherapie bei Krebspatienten.

Letztendlich kann die Schlußfolgerung gezogen werden, daß die Viscumpräparation in oraler Applikationsform therapeutisch als Adjuvanz der malignen Tumorbehandlung sowie der chemozytostatischen und radiologischen Tumorbehandlung geeignet ist.

Obwohl unterschiedliche sekundäre Surrogate das pharmakodynamische Wirkprofil extraktspezifisch beschreiben, ist ein "Grundschema allgemeiner Reaktionen" in der Wirkung zu erkennen. In Abhängigkeit von Wirkstoff, Dosis und Dauer der Applikation wurden Targetorgane aufgrund von Wechselwirkungen zwischen Endokrinum, Nerven- und Immunsystem beeinflußt.

## Patentansprüche

1. Verwendung von standardisierten **Mistel-(Viscum album)-Trockenextrakten** mit einem Droge-Extrakt-Verhältnis (DEV nativ) von 4 zu 1 bis 7 zu 1, entsprechend 20 zu 1 bis 35 zu 1 bei der Frischpflanze zur Herstellung von oral applizierbaren Arzneimitteln zur Behandlung von allgemein immunsupprimierten Zuständen und zur zellulären Immunstimulation.

2. Verwendung nach Anspruch 1 zur Behandlung von strahlen-, infektions- und chemisch bedingten Organ- und Gewebeschädigungen, insbesondere des O-MALT-Systems des Dünndarms (entzündliche Erkrankungen, Enteritis regionalis Crohn), des Knochenmarks (Knochenmarkaplasie), des Thymus (Dysfunktion, Aplasie oder Hypoplasie), der Milz (Dysfunktion) und/oder der Lymphknoten (Aplasie oder Hypoplasie infolge medikamentenoder strahlenbedingter Schädigung), der Leber (Atrophie, Nekrose), Hepatitis A, B und C, der Bauchspeicheldrüse (Insuffizienz der exokrinen, sekretorischen Funktion von Proteasen, Esterasen, Carbohydrasen und/oder Nukleasen sowie der endokrinen Funktionsstörung des Kohlenhydratstoffwechsels [Langerhanssche Inseln]) und/oder der Nieren (Insuffizienz), zur Therapie von Leukozytopenie, Granulozytopenie, Lymphozytopenie, Thrombozytopenie, Erythrozytopenie und bei Immunglobulinmangelzuständen, auch infolge von AIDS und/oder Tumoren sowie zur Therapie von Hyperlipoproteinämien, Hyperproteinämie und Hyperlipidämie.

3. Verwendung nach Anspruch 1, **dadurch gekennzeichnet, daß** die zelluläre Immunstimulation eine Zunahme der Zellzahlen von Leukozyten, Thrombozyten, Erythrozyten, segmentkernigen Granulozyten und Lymphozyten, T-, B-, Helfer-, Suppressor- und NK-Zellen, insbesondere von B-Lymphozyten in der terminalen Strombahn bewirkt, daß eine Zunahme vitaler Lymphozyten in der Rindenzone und den -follikeln in den Peyersche Plaques (Dünndarm) zu erkennen ist, die mit einer sekretorischen Induktion von sIGA in den Dünndarm verbunden ist, daß eine Zunahme aller Blutbildungselemente im Knochenmark feststellbar ist, daß eine zelluläre Stimulation immunkompetenter Zellen in Thymus, Milz und mesenterialen Lymphknoten nachweisbar ist und/oder daß die Stoffwechselfunktionen von Leber, Pankreas und Nieren (Senkung der Serumwerte von Kreatinin, Harnsäure, Glukose, GOT, GPT und GGT) deutlich aktiviert werden.

4. Verwendung nach Anspruch 1 zur Behandlung von Störungen des Kohlenhydratstoffwechsels und/oder der Leber- und Nierenfunktion, zur Behandlung von prädiabetischen, insbesondere senilen Verlaufsformen, zur Behandlung von latenter diabetischer Stoffwechsellage, insbesondere bei Schwangerschaft, zur Behandlung von Infektionen, Streß und/oder Fettleibigkeit, zur Behandlung von Bluthochdruck als synergistisches Adjuvanz nach simultaner Applikation mit chemisch definierten Antihypertonika und als adjuvante Therapie der Diabetes mellitus bei vorhandener Restfunktion der Pankreas.

5. Verwendung nach Anspruch 1 als orale Adjuvanzien der malignen Tumorbehandlung, - insbesondere der Behandlung von Mamma-, Portio-, Kolon- oder Prostatakarzinomen, auch in Kombination mit Cytostatika, insbesondere Cyclophosphamid oder Strahlentherapie -, der Behandlung von Erkrankungen mit supprimiertem Immunsystem, insbesondere AIDS.

6. Verwendung nach Anspruch 1 zur Steigerung der Verträglichkeit von chemo-, zytostatischer, radiologischer Therapie, insbesondere in Kombination mit chemisch definierten Substanzen, ausgewählt aus Clofibrat, ACE-Hemmern, a-2-Rezeptor-Antagonisten, Ca-Antagonisten und/oder Diuretika, als Adjuvanz der Analgesie, insbesondere als pharmakologische Komponente der positiven Beeinflussung der Wechselwirkungen zwischen Endokrinum, Nerven- und Immunsystem.

7. Verwendung nach Anspruch 1, als Adujvanz bei der Behandlung von bakteriell und viral induzierten Krankheitsbildern, insbesondere entzündlichen Erkrankungen des Dünndarms (Enteritis regionalis Crohn), der Bauchspeicheldrüse und der Nieren, Leberatrophie, Hepatitis A, B und C, Hautläsionen (Ulcus cruris), wie auch Herpes simplex I und II und Herpes zoster.

8. Verwendung nach Anspruch 1 zur Behandlung von Hyperlipoproteinämien, insbesondere Hypercholesterinämie und/oder Hypertriglyceridämie.

9. Verwendung nach Anspruch 1 zur Behandlung von hämorrhagischen Störungen, insbesondere Metrorrhagie oder hormonellen Dystinktionen, insbesondere Amenorrhoe und Dysmenorrhoe, zur Senkung des erhöhten Blutungsrisikos, insbesondere infolge der gegebenenfalls simultanen Einnahme von Ca-Antagonisten und rekombinatem Gewebe-Plasminogenaktivator (PA), zur Behandlung von Anämie - insbesondere bei Tumorpatienten, - zur Abnahme des Plasma-Noradrenalinwertes, als Adjuvanz der Analgesie- und Tinnitus-Behandlung, insbesondere als pharmakologische Komponente der positiven Beeinflussung der Wechselwirkungen zwischen Endokrinum, Nerven- und Immunsystem.

10. Verwendung nach einem der Ansprüche 1 bis 8 als Frischpflanze, insbesondere Preßsaft in extrahierter Form mit Hilfe von wäßrigen, organischen und/oder überkritischen Lösungmitteln, insbesondere Kohlendioxid, in getrockneter Form, insbesondere Pulver, Trockenextrakte aus der Frischpflanze oder Droge (getrocknete Pflanze), Granulat, insbesondere Kapsel und als Tablette, insbesondere Dragee oder Pastille.

11. Oral applizierbares Arzneimittel zur Behandlung von strahlen-, infektions- und chemisch bedingten Organ- und Gewebeschädigungen, insbesondere des O-MALT-Systems des Dünndarms (entzündliche Erkrankungen, Enteritis regionalis Crohn), des Knochenmarks (Knochenmarkaplasie), des Thymus (Dysfunktion, Aplasie oder Hypoplasie), der Milz (Dysfunktion) und der Lymphknoten (Aplasie oder Hypoplasie infolge medikamenten- oder strahlenbedingter Schädigung), der Leber (Atrophie, Nekrose), Hepatitis A, B und C, der Bauchspeicheldrüse (Insuffizienz der exokrinen, sekretorischen Funktion von Proteasen, Esterasen, Carbohydrasen und Nukleasen sowie der endokrinen Funktionsstörung des Kohlenhydratstoffwechsels [Langerhanssche Inseln]) und der Nieren (Insuffizienz), zur Therapie von Leukozytopenie, Granulozytopenie, Lymphozytopenie, Thrombozytopenie, Erythrozytopenie und bei Immunglobulinmangelzuständen, auch infolge von AIDS und Tumoren sowie zur Therapie von Hyperlipoproteinämien, Hyperproteinämie und Hyperlipidämie enthaltend Mistel-(Viscum)-Extrakte in Kombination mit Echinacea-Extrakten, insbesondere Trockenextrakten (E. pallida und E. angustifolia), Artischocken-(Cynara)-Extrakten und/oder mit Brennessel-(Urtica)-Extrakten, insbesondere Trockenextrakten aus den Wurzeln, Blättern oder Kraut.

12. Arzneimittel nach Anspruch 11, enthaltend Echinacea-Extrakte, mit einem Droge-Extrakt-Verhältnis von 4 zu 1 bis 8 zu 1.

13. Arzneimittel nach Anspruch 12, enthaltend Artischocken-(Cynara)-Extrakte mit einem Droge-Extraktverhältnis von 4 zu 1 bis 8 zu 1, entsprechend 25 zu 1 bis 35 zu 1 bei der Frischpflanze.

14. Arzneimittel nach Anspruch 11, enthaltend Brennessel-(Urtica)-Extrakte mit einem Droge-Extrakt-Verhältnis von 6 zu 1 bis 15 zu 1.

15. Oral applizierbares Arzneimittel nach einem oder mehreren der Ansprüche 11 bis 14 in Calciumcarbonat- und Saccharid-haltiger Granulatform.

## Claims

1. Use of standardized mistletoe (Viscum album) dry extracts having a drug-to-extract ratio (DEV native) of from 4:1 to 7:1, corresponding to 20:1 to 35:1 for the fresh plant, for the preparation of orally administered medicaments for the treatment of generally immunosuppressed conditions and for cellular immunostimulation.

2. The use according to claim 1 for the treatment of organ and tissue damages caused by radiation, infections and chemically, especially of the O-MALT system of the small intestine (inflammatory diseases, Enteritis regionalis Crohn), of the bone marrow (bone marrow aplasia), thymus (dysfunction, aplasia or hypoplasia), spleen (dysfunction) and the lymph nodes (aplasia or hypoplasia due to damage from medicaments or radiation), liver (atrophy, necrosis), hepatitis A, B and C, pancreas (insufficiency of the exocrinic secretory function of proteases, esterases, carbohydrases and nucleases, and the endocrinic functional disorder of carbohydrate metabolism [Langerhans islets]) and the kidneys (insufficiency), for the therapy of leucocytopenia, granulocytopenia, lymphocytopenia, thrombocytopenia, erythrocytopenia, and in immunoglobulin deficiencies, also due to AIDS and tumors, and for the therapy of hyperlipoproteinemias, hyperproteinemia and hyperlipidemia.

3. The use according to claim 1, **characterized in that** the cellular immunostimulation causes an increase of the cell counts of leucocytes, platelets, erythrocytes, polymorphonuclear granulocytes and lymphocytes, T, B, helper, suppressor and NK cells, especially B lymphocytes, in the terminal vascular system, that an increase in vital lymphocytes can be seen in the cortical zone and follicles in Peyer's plaques (small intestine) which is associated with a secretory induction of sIGA into the small intestine, that an increase of all blood forming elements can be seen in the bone marrow, that a cellular stimulation of immunocompetent cells can be detected in the thymus, spleen and mesenteric lymph nodes, and/or that the metabolic functions of the liver, pancreas and kidneys (reduction of the serum levels of creatinin, uric acid, glucose, GOT, GPT and GGT) are clearly activated.

4. The use according to claim 1 for the treatment of disorders of carbohydrate metabolism and of liver and kidney functions, for the treatment of prediabetic, especially senile forms, for the treatment of latent diabetic metabolic condition, especially in pregnancy, for the treatment of infections, stress and/or obesity, for the treatment of blood hypertension as a synergistic adjuvant after simultaneous administration of chemically defined antihypertensive agents, and as an adjuvant therapy of Diabetes mellitus when there is some residual function of the pancreas.

5. The use according to claim 1 as oral adjuvants for the treatment of malignant tumors, especially for the treatment of carcinomas of the mamma, cervix, colon or prostate gland, also in combination with cytostatics, especially cyclophosphamide, or radiation therapy, and for the treatment of diseases with suppressed immune system, especially AIDS.

6. The use according to claim 1 for increasing the tolerance for chemotherapy, cytostatic or radiologic therapies, especially in combination with chemically defined substances selected from clofibrate, ACE inhibitors, α-2 receptor antagonists, Ca antagonists and/or diuretics, as an analgetic adjuvant, especially as a pharmacological component having a positive influence on the interactions between the endocrine, nervous and immune systems.

7. The use according to claim 1 as an adjuvant for the treatment of diseases induced by bacteria and viruses, especially inflammatory diseases of the small intestine (Enteritis regionalis Crohn), pancreas and kidneys, liver atrophy, hepatitis A, B and C, skin lesions (Ulcus cruris), and also Herpes simplex I and II, and Herpes zoster.

8. The use according to claim 1 for the treatment of hyperlipoproteinemias, especially hypercholesterolemia and/or hypertriglyceridemia.

9. The use according to claim 1 for the treatment of hemorrhagic disorders, especially metrorhagia or hormonal dystinctions, especially amenorrhea and dysmenorrhea, for reducing increased risk of bleeding, especially due to the optional simultaneous intake of Ca antagonists and recombinant tissue plasminogen activator (PA), for the treatment of anemia, especially in tumor patients, for decreasing the plasma noradrenalin level, as an adjuvant in analgetics and tinnitus treatment, especially as a pharmacological component having a positive influence on the interactions between the endocrine, nervous and immune systems.

10. The use according to any of claims 1 to 8 as a fresh plant, especially extracted juice as extracted using aqueous, organic and/or supercritical solvents, especially carbon dioxide, in a dried form, especially powder, dry extracts from the fresh plant or drug (dried plant), granules, especially capsule, and as a tablet, especially coated tablet, or pastille.

11. An orally administered medicament for the treatment of organ and tissue damages caused by radiation, infections and chemically, especially of the O-MALT system of the small intestine (inflammatory diseases, Enteritis regionalis Crohn), of the bone marrow (bone marrow aplasia), thymus (dysfunction, aplasia or hypoplasia), spleen (dysfunction) and the lymph nodes (aplasia or hypoplasia due to damage from medicaments or radiation), liver (atrophy, necrosis), hepatitis A, B and C, pancreas (insufficiency of the exocrinic secretory function of proteases, esterases, carbohydrases and nucleases, and the endocrinic functional disorder of carbohydrate metabolism [Langerhans islets]) and the kidneys (insufficiency), for the therapy of leucocytopenia, granulocytopenia, lymphocytopenia, thrombocytopenia, erythrocytopenia, and in immunoglobulin deficiencies, also due to AIDS and tumors, and for the therapy of hyperlipoproteinemias, hyperproteinemia and hyperlipidemia, containing mistletoe (Viscum) extracts in combination with Echinacea extracts, especially dry extracts *(E. pallida* and *E. angustifolia),* artichoke (Cynara) extracts and/or with nettle (Urtica) extracts, especially dry extracts from the roots, leaves or herbage.

12. The medicament according to claim 11, containing Echinacea extracts with a drug-to-extract ratio of from 4:1 to 8:1.

13. The medicament according to claim 12, containing artichoke (Cynara) extracts with a drug-to-extract ratio of from 4:1 to 8:1, corresponding to 25:1 1 to 35:1 for the fresh plant.

14. The medicament according to claim 11, containing nettle (Urtica) extracts with a drug-to-extract ratio of from 6:1 to 15:1.

15. An orally administered medicament according to one or more of claims 11 to 14 in the form of granules containing calcium carbonate and saccharide.

## Revendications

1. Emploi d'un extrait sec standardisé de gui (Viscum album) présentant un rapport drogue/extrait (RDEₙₐₜ) de 4/1 à 7/1, ce qui correspond à une valeur de 20/1 à 35/1 pour des matières végétales fraîches, dans la préparation d'un médicament à administrer par voie orale, pour le traitement des états d'immunodépression en général et pour l'immunostimulation cellulaire.

2. Emploi conforme à la revendication 1, pour le traitement de lésions provoquées par des rayons, une infection ou un agent chimique dans des organes ou des tissus, en particulier au niveau du système MALT (tissu lymphoïde associé aux muqueuses) organisé de l'intestin grêle (maladies inflammatoires, entérite régionale ou maladie de Crohn), de la moelle osseuse (aplasie médullaire), du thymus (dysfonctionnement, aplasie ou hypoplasie), de la rate (dysfonctionnement), des ganglions lymphatiques (aplasie ou hypoplasie consécutive à une lésion provoquée par des médicaments ou des rayons), du foie (atrophie, nécrose, hépatite A, B ou C) du pancréas (insuffisance de la fonction de sécrétion exocrine de protéases, d'estérases, de carbohydrases et/ou de nucléases, ainsi que troubles de la fonction endocrine d'échange des sucres (îlots de Langerhans)) et/ou des reins (insuffisance), pour le traitement de leucocytopénies, de granulocytopénies, de lymphocytopénies, de thrombocytopénies, d'érythrocytopénies ou d'états de déficit d'immunoglobulines, aussi par suite de sida et/ou de tumeur, ou pour le traitement d'hyperlipoprotéinémies, d'hyperprotéinémies ou d'hyperlipidémies.

3. Emploi conforme à la revendication 1, **caractérisé en ce que** l'immunostimulation cellulaire entraîne une augmentation des numérations cellulaires en leucocytes, thrombocytes, érythrocytes, granulocytes à noyau segmenté et lymphocytes B, T, auxiliaires, suppresseurs et NK, en particulier des lymphocytes B dans les vaisseaux terminaux, **en ce qu'**on peut déceler une augmentation des lymphocytes vitaux au niveau de la zone corticale et des follicules des plaques de Peyer de l'intestin grêle, qui est associée à une induction de la sécrétion de s-IgA dans l'intestin grêle, **en ce qu'**on peut établir une augmentation de tous les constituants du sang dans la moelle osseuse, **en ce qu'**on peut détecter une stimulation cellulaire des cellules immunocompétentes dans le thymus, la rate et les ganglions lymphatiques mésentériques, et/ou **en ce que** les fonctions métaboliques du foie, du pancréas et des reins (diminution des taux sériques de créatinine, d'acide urique, de glucose, de GOT, de GPT et de GGT) sont nettement activées.

4. Emploi conforme à la revendication 1, pour le traitement des troubles du métabolisme des sucres et/ou des fonctions hépatique et rénale, pour le traitement des formes évolutives prédiabétiques, en particulier séniles, pour le traitement des conditions métaboliques de diabète latent, en particulier en cas de grossesse, pour le traitement des infections, du stress et/ou de l'obésité, pour le traitement de l'hypertension, comme adjuvant synergique après administration simultanée d'un antihypertenseur chimique défini, et comme traitement adjuvant du diabète sucré en présence d'une fonction pancréatique résiduelle.

5. Emploi conforme à la revendication 1, comme adjuvant oral du traitement de tumeurs malignes, en particulier des carcinomes du sein, de l'utérus, du côlon ou de la prostate, aussi en combinaison avec des cytostatiques, en particulier un cyclophosphamide, ou une radiothérapie, ou du traitement de maladies où il y a suppression du système immunitaire, en particulier du sida.

6. Emploi conforme à la revendication 1, pour augmenter la compatibilité d'une chimiothérapie, d'une thérapie par cytostatique ou d'une radiothérapie, en particulier en combinaison avec des substances chimiquement définies, choisies parmi le clofibrate, les inhibiteurs de l'ACE, les antagonistes des récepteurs α2, les antagonistes du calcium et/ou les diurétiques, ou comme adjuvant d'analgésie, en particulier comme composante pharmacologique de l'influence positive des interactions entre le système endocrinien, le système nerveux et le système immunitaire.

7. Emploi conforme à la revendication 1, comme adjuvant dans le traitement de syndromes à induction bactérienne ou virale, en particulier de maladies inflammatoires de l'intestin grêle (entérite régionale ou maladie de Crohn), du pancréas ou des reins, de l'atrophie hépatique, des hépatites A, B et C, de lésions cutanées (ulcus cruris), ou aussi de l'herpes simplex I ou II et de l'herpes zoster.

8. Emploi conforme à la revendication 1, pour le traitement des hyperlipoprotéinémies, en particulier de l'hypercholestérolémie et/ou de l'hypertriglycéridémie.

9. Emplois conforme à la revendication 1, pour le traitement des troubles hémorragiques, en particulier des métrorragies, ou des dérèglements hormonaux, en particulier de l'aménorrhée ou de la dysménorrhée, pour faire baisser un risque élevé de saignement, en particulier à la suite d'une prise éventuellement simultanée d'un antagoniste du calcium et d'activateur tissulaire du plasminogène recombinant, pour le traitement de l'anémie, en particulier chez des patients atteints de tumeurs, pour faire baisser le taux plasmatique de noradrénaline, comme adjuvant du traitement de l'analgésie ou du bourdonnement d'oreilles, en particulier comme composante pharmacologique de l'influence positive des interactions entre le système endocrinien, le système nerveux et le système immunitaire.

10. Emploi, conforme à l'une des revendications 1 à 8, à l'état de matières végétales fraîches, en particulier de jus pressé sous forme d'extrait obtenu à l'aide de solvants aqueux, organiques ou supercritiques, en particulier de dioxyde de carbone, sous forme séchée, en particulier de poudre, d'extrait sec de matières végétales fraîches ou de drogue (matières végétales séchées), de granulat, en particulier de capsules, ou à l'état de comprimés, en particulier de dragées ou de pastilles.

11. Médicament administrable par voie orale, destiné au traitement de lésions provoquées par des rayons, une infection ou un agent chimique dans des organes ou des tissus, en particulier au niveau du système MALT (tissu lymphoïde associé aux muqueuses) organisé de l'intestin grêle (maladies inflammatoires, entérite régionale ou maladie de Crohn), de la moelle osseuse (aplasie médullaire), du thymus (dysfonctionnement, aplasie ou hypoplasie), de la rate (dysfonctionnement), des ganglions lymphatiques (aplasie ou hypoplasie consécutive à une lésion provoquée par des médicaments ou des rayons), du foie (atrophie, nécrose, hépatite A, B ou C) du pancréas (insuffisance de la fonction de sécrétion exocrine de protéases, d'estérases, de carbohydrases et de nucléases, ainsi que troubles de la fonction endocrine d'échange des sucres (îlots de Langerhans)) et des reins (insuffisance), pour le traitement de leucocytopénies, de granulocytopénies, de lymphocytopénies, de thrombocytopénies, d'érythrocytopénies et d'états de déficit d'immunoglobulines, aussi par suite de sida ou de tumeurs, ou pour le traitement d'hyperlipoprotéinémies, d'hyperprotéinémies et d'hyperlipidémies, lequel médicament contient de l'extrait de gui (Viscum) en combinaison avec des extraits d'échinacée, en particulier des extraits secs (E. pallida et E. angustifolia), des extraits d'artichaut (Cynara) et/ou des extraits d'ortie (Urtica), en particulier des extraits secs de racines, de feuilles ou de tiges.

12. Médicament conforme à la revendication 11, contenant des extraits d'éricacée présentant un rapport drogue/extrait de 4/1 à 8/1.

13. Médicament conforme à la revendication 12, contenant des extraits d'artichaut (Cynara) présentant un rapport drogue/extrait de 4/1 à 8/1, correspondant à une valeur de 20/1 à 35/1 pour les matières végétales fraîches.

14. Médicament conforme à la revendication 11, contenant des extraits d'ortie (Urtica) présentant un rapport drogue/extrait de 6/1 à 15/1.

15. Médicament administrable par voie orale, conforme à l'une ou à plusieurs des revendications 11 à 14, se présentant sous forme de granulés contenant du carbonate de calcium et un saccharide.
